# EUROPÄISCHE PATENTSCHRIFT

(11) **EP 0 619 990 B1**
(45) Veröffentlichungstag und Bekanntmachung des Hinweises auf die Patenterteilung: **02.06.1999**
(21) Anmeldenummer: 93810273.8
(22) Anmeldetag: 15.04.1993
(51) Int. Cl.: A61F 2/34

(54) **Bausatz für eine künstliche Gelenkspfanne, insbesondere eine Hüftgelenkspfanne**
Kit for an artificial joint cup, especially an acetabular cup
Jeu de pièces pour une cupule articulaire artificielle, notamment une cupule acétabulaire

(43) Veröffentlichungstag der Anmeldung: 19.10.1994
(73) Patentinhaber: Sulzer Orthopädie AG, 6340 Baar (CH)
(72) Erfinder: Frei, Heribert, CH-8200 Schaffhausen (CH)
(74) Vertreter: Trieblnig, Adolf

(56) Entgegenhaltungen:
- EP-A- 0 314 951
- EP-A- 0 499 475
- DE-A- 3 205 526
- DE-A- 3 310 944

## Beschreibung

Die Erfindung betrifft einen Bausatz für eine künstliche Gelenkspfanne, insbesondere eine Hüftgelenkspfanne, entsprechend den Merkmalen des Patentanspruchs 1.

Ein aus der US-Patentschrift 5,021,062 bekannter Bausatz der genannten Art enthält eine in das Knochengewebe einschraubbare Knochenschraube oder einen Knochennagel als Befestigungselement, welches von innen her durch die Führungsbohrung in das Knochengewebe eingebracht und schliesslich mit einem an einem Kopfteil ausgebildeten Gewindeabschnitt in die Aussenschale eingeschraubt wird. Die Knochenschraube ist mit einem in das Knochengewinde einschraubbaren zweiten Gewindeabschnitt ausgeführt, dessen Aussendurchmesser und dessen Steigung grösser sind als der Aussendurchmesser und die Steigung des in die Führungsbohrung der Aussenschale einschraubbaren ersten Gewindeabschnitts, wobei der Kerndurchmesser und die Gewindeform des zweiten Gewindeabschnitts so gewählt sind, dass zwei aufeinanderfolgende Gewindegänge dieses zweiten Gewindeabschnitts über zwei aufeinanderfolgende Gewindegänge der Führungsbohrung geführt werden können, ohne in diese einzugreifen. Entsprechend können die Führungsbohrungen der Aussenschale nur mit je höchstens zwei für die Verschraubung des Befestigungselementes wirksamen Gewindegängen ausgeführt werden, um ein unbehindertes Einschrauben der Knochenschraube in das Knochengewebe zu ermöglichen. Bei der bekannten Anordnung stehen somit nur diese relativ kurzen Gewindeabschnitte für die Uebertragung der jeweils auftretenden, unter Umständen beträchtlichen Stützkräfte und für die Sicherung der entsprechend hoch beanspruchten Schraubverbindung zwischen der Knochenschraube und der Aussenschale zur Verfügung. Der bekannte Bausatz ist daher für Anwendungen, bei denen die anatomischen Verhältnisse im Implantationsbereich innerhalb relativ weiter Grenzen variieren können, z.B. als Ersatz für einen fehlenden Beckenteil im Bereich des Hüftgelenks, nur in beschränktem Masse geeignet, da in manchen derartigen Fällen eine sichere Befestigung der Gelenkpfanne durch Knochenschrauben nicht möglich ist, wobei eine relativ aufwendige Lagerhaltung eines Sortiments von mehreren, in unterschiedlichen Längen ausgeführten Knochenschrauben erforderlich ist, um eine ständige Verfügbarkeit von Bausätzen für derartige, relativ seltene Anwendungen zu gewährleisten.

Ein aus der EP-A-0 314 951 bekannter Bausatz einer anderen Gattung enthält eine innerhalb eines Hohlraums des Knochengewebes angeordnete, mit Gewindebohrungen versehene Hüftgelenk-Pfannenprothese mit in die Gewindebohrungen einschraubbaren, von der Pfanneninnenseite nach aussen vorstreckbaren Stützen, die an ihren knochenseitigen Enden je mit einem an die Wandung des Hohlraums anlegbaren Stützteller versehen sein können. Diese Stützen sind als Distanzhalter zur Ueberbrückung des Abstandes zwischen der Prothesenoberfläche und der durch die Wandung des die Prothesenpfanne umgebenden Hohlraums gebildeten Knochenoberfläche und zur Abstützung der Prothesenpfanne an den Wänden dieses Hohlraums vorgesehen. Die Distanzhalter dieser Ausführung sind zur permanenten Befestigung der Pfannenprothese am Knochengewebe nicht vorgesehen und auch nicht geeignet; sie sollen vielmehr lediglich während der Rekonstruktion des Knochengewebes im Hohlraum eine primäre Funktions- und Uebungsstabilität gewährleisten, bis sich das neu gebildete Knochengewebe nach einigen Wochen bis Monaten an der Kraftübertragung auf die Prothese beteiligt.

Der Erfindung liegt die Aufgabe zugrunde, einen weiter entwickelten Bausatz der eingangs genannten Art in einer Bauweise zu schaffen, welche eine gegenüber bisherigen Ausführungen günstigere Beanspruchung des Knochengewebes und der zusammenwirkenden Teile gewährleistet und welche eine sichere Uebertragung der zwischen dem Knochengewebe und der Aussenschale wirkenden Stützkräfte mit einfach herstellbaren und auf einfache Weise verstellbaren Bauteilen gestattet, die mit geringem Arbeits- und Zeitaufwand an unterschiedliche anatomische Verhältnisse angepasst und bezüglich des Implantationsbereichs positioniert werden können.

Diese Aufgabe wird gemäss der Erfindung durch die im Patentanspruch 1 angegebenen Merkmale gelöst.

Der erfindungsgemäss ausgeführte Bausatz gestattet eine von den Durchmessern der in der Aussenschale angebrachten Führungsbohrungen unabhängige, an den Durchmesser der Verankerungsbohrung angepasste Bemessung der Stützplatte des Befestigungselementes. Die mit einer relativ grossen wirksamen Abstützfläche ausführbare Stützplatte kann mit der innerhalb der Verankerungsbohrung in einer vorbestimmten Tiefe anbringbaren stirnseitigen Anpressfläche zusammengeführt werden, welche die Uebertragung der jeweils auftretenden Stützkräfte unter minimaler Druckbeanspruchung des Knochengewebes ermöglicht. Der erfindungsgemäss ausgeführte Bausatz gestattet ferner auf einfache Weise eine Anpassung der Abstützlänge des Befestigungselementes an unterschiedliche anatomische Verhältnisse, z.B. im Falle einer erforderlichen Ueberbrückung eines fehlenden bzw. zu ersetzenden Teils des Knochens, so dass eine genaue Positionierung der Gelenkspfanne ausserhalb des Knochengewebes erzielbar ist. Ein weiterer Vorteil des erfindungsgemäss ausgeführten Bausatzes besteht darin, dass die Aussenschale vorgängig, ausserhalb des Implantationsbereichs, mit dem die vorbestimmte Abstützlänge aufweisenden Befestigungselement - oder mit mehreren entsprechenden Befestigungselementen - bestückt werden kann, wobei der Stützteil von der Aussenseite her in die Aussenschale eingeführt und mit dem von der Innenseite her in die Führungsbohrung einschraubbaren Kopfteil lösbar verbunden werden kann. Hierauf kann die Aussenschale in fertig montiertem Zustand, mit gegen die Mitte der Aussenschale hin eingezogenen Befestigungselementen und an die Aussenseite der Aussenschale anliegenden Stützplatten, als zusammenhängende, kompakte Baueinheit in den Implantationsbereich eingesetzt und auf die Verankerungsbohrungen im Knochengewebe ausgerichtet werden. Entsprechend kann das Befestigungselement durch eine einfache Verschiebebewegung radial in die Verankerungsbohrung eingeführt und durch Verschrauben des Kopfteils mit der Aussenschale positioniert werden. Der in der Führungsbohrung fest verschraubbare Kopfteil ermöglicht dabei eine robuste, biegesteife Verbindung zwischen der Aussenschale und dem Befestigungselement.

Ausgestaltungen des Erfindungsgegenstandes sind in den abhängigen Patentansprüchen angegeben.

Weitere Merkmale und Einzelheiten ergeben sich aus der folgenden Beschreibung eines in der Zeichnung schematisch dargestellten Ausführungsbeispiels der Erfindung, in Verbindung mit den Patentansprüchen. In der Zeichnung zeigen:
- Fig.1: Teile eines erfindungsgemäss ausgebildeten Bausatzes in einem bezüglich der Aussenschale diametral verlaufenden Schnitt;
- Fig.2: weitere Teile des Bausatzes in einem Schnitt entsprechend der Linie II-II in Fig.1;
- Fig.3: einen als Hilfsvorrichtung zum Vorbereiten eines Implantationsbereiches vorgesehenen weiteren Teil des Bausatzes in einem der Fig. 1 entsprechenden Schnitt;
- Fig. 4: eine Einzelheit des Bausatzes nach Fig. 1 in einer grösseren Darstellung.

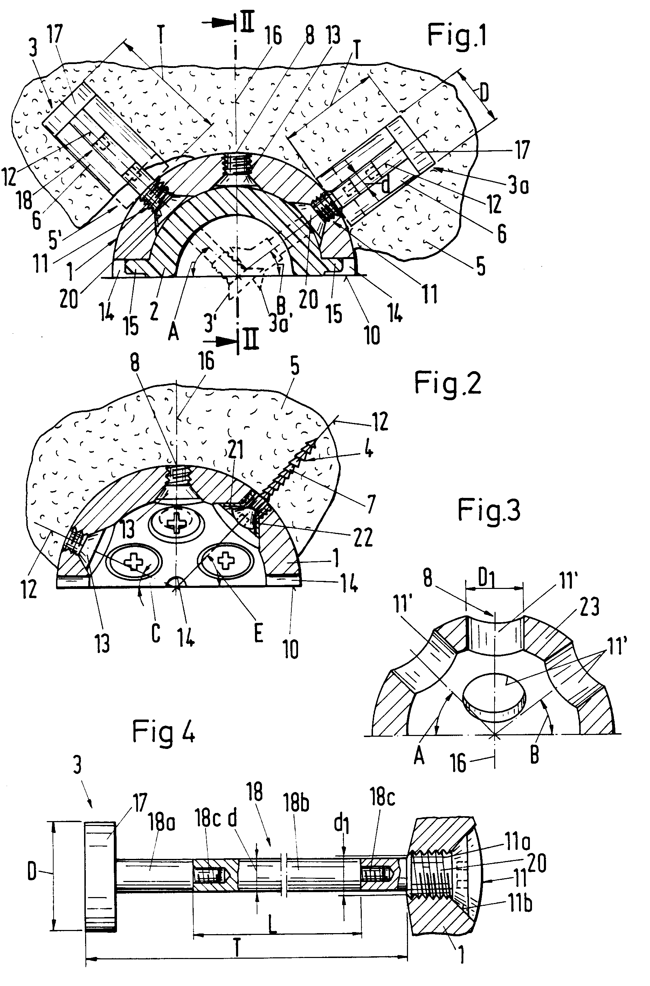

Der Bausatz nach den Figuren 1 und 2 enthält eine metallische Aussenschale 1 in Form einer hohlen Halbkugel, eine in diese einsetzbare, ebenfalls halbkugelförmig ausgeführte Innenschale 2 und an der Aussenschale 1 radial abstehend anbringbare Befestigungselemente 3, 3a und 4, welche im Knochengewebe 5 eines menschlichen Beckenteils befestigbar sind und durch welche die Aussenschale 1 ausserhalb des Knochengewebes 5 positionierbar ist. Der dargestellte Bausatz ist als Ersatz für einen fehlenden Beckenteil im Bereich des Hüftgelenks vorgesehen. Die Befestigungselemente 3 und 3a sind je in Form eines in einer Verankerungsbohrung 6 des Knochengewebes abstützbaren Sockels ausgeführt. Das zusätzliche Befestigungselement 4 kann in Form eines Knochennagels oder, gemäss Fig. 2, als in eine weitere Verankerungsbohrung des Knochengewebes 5 einschraubbare Knochenschraube 7 ausgeführt sein kann.

Die Aussenschale 1 ist im Bereich ihres Pols 8 mit einer radialen Führungsbohrung 11 versehen, welche einen äusseren Gewindeabschnitt 11a und einen zur Aufnahme eines Schraubenkopfes geeigneten, bei der dargestellten Ausführung kegelförmigen, inneren Abschnitt 11b aufweist. Entsprechende weitere radiale Führungsbohrungen 11 sind zwischen dem Pol 8 und dem Aequator 10 der Aussenschale 1 in Umfangsrichtung gegeneinander versetzt, mit zur Ebene des Aequators 10 in unterschiedlichen Winkeln A, B, C und D angeordneten, geneigten Achsen 12 ausgeführt. Die Führungsbohrungen 11 sind je zur Aufnahme eines der Befestigungselemente 3, 3a bzw. 4 oder einer Verschlussschraube 13 bestimmt. Anstelle der dargestellten Ausführung mit zwei Befestigungselementen 3, 3a sind auch Ausführungen mit nur einem oder mit mehreren entsprechenden Befestigungselementen 3, 3a möglich. Ebenso können mehrere zusätzliche Befestigungselemente 4 vorgesehen sein. Gegebenenfalls kann auf das zusätzliche Befestigungselement 4 auch verzichtet werden.

Die Aussenschale 1 kann im Bereich des Aequators 10 mit mehreren, z.B. acht, über den Umfang verteilten Ausnehmungen 14 ausgeführt sein, die zur Aufnahme von an der Innenschale 2 vorgesehenen Nocken 15 bestimmt sind. Die Innenschale 2, die ebenfalls metallisch oder, wie dargestellt, aus einem Kunststoff ausgeführt sein kann, ist über die Nocken 15 in unterschiedlichen Winkelstellungen um eine durch den Pol 8 verlaufende Polachse 16 mit der Aussenschale 1 verklinkbar.

Die sockelförmigen Befestigungselemente 3 und 3a enthalten je eine kreisrunde Stützplatte 17, einen jeweils von aussen in eine der Führungsbohrungen 14 einführbaren stengelartigen Stützteil 18 und einen jeweils von der Innenseite der Aussenschale 1 in eine der Führungsbohrungen 11 einschraubbaren Kopfteil 20. Die Stützplatte 17 ist mit einem Durchmesser D ausgeführt, der wesentlich grösser ist als der Durchmesser d des Stützteils 18, welcher seinerseits um ein vorbestimmtes Spiel kleiner ist als der Durchmesser d₁ des Gewindeabschnitts 11a der Führungsbohrung 11, der dem Kerndurchmesser des Gewindeabschnitts 20a des Kopfteils 20 entspricht. Der Stützteil 18 ist in der Führungsbohrung 11 lose radial verschiebbar angeordnet und mit dem von innen her in die Führungsbohrung 11 einschraubbaren Kopfteil 20 über einen in eine stirnseitige Gewindebohrung des Kopfteils 20 einschraubbaren Gewindezapfen 18c lösbar verbunden. Der Stützteil 18 kann einstückig ausgeführt oder aus mehreren, darstellungsgemäss zwei Teilstücken 18a und 18b zusammengesetzt sein, wobei das eine Teilstück 18a mit der Stützplatte 17 fest verbunden und mit dem zweiten Teilstück 18b ebenfalls über einen Gewindezapfen 18c lösbar verbunden ist. Eine lösbare Schraubverbindung mit einem entsprechenden, nicht dargestellten Gewindezapfen kann auch zwischen der Stützplatte 17 und dem Stützteil 18 bzw. dem Teilstück 18a vorgesehen sein. Abweichend von der dargestellten Ausführung, kann sich der Stützteil 18 bzw. das Teilstück 18b bis zum Gewindeabschnitt des Kopfteils 20 erstrecken, wobei der betreffende Gewindezapfen 18c sich in eine im Gewindeabschnitt oder, wie dargestellt, in einem Fortsatz des Gewindeabschnitts ausgebildete Gewindebohrung erstrecken kann. Entsprechende Schraubverbindungen können auch mit vom Kopfteil 20 gegen das Teilstück 18b und mit von diesem gegen das Teilstück 18a abstehenden Gewindezapfen 18c ausgeführt sein.

Zweckmässig ist es, wenn dem Chirurgen ein Vorrat aus mehreren Stützteilen 18, Teilstücken 18a oder, wie beim dargestellten Beispiel, aus den einfachen Teilstücken 18b mit unterschiedlichen, z.B. millimeterweise abgestuften Längenabmessungen L zur Verfügung steht, so dass bei relativ geringem Lageraufwand ein rascher Zugriff zu den den jeweils vorliegenden anatomischen Verhältnissen entsprechend auszuwählenden und gegebenenfalls miteinander zu kombinierenden Teilstücken 18b gewährleistet werden kann. Bei der Ausführung nach Fig. 1 sind die Befestigungselemente 3 und 3a mit unterschiedlichen Abstützlängen ausgeführt, wobei das die grössere Abstützlänge aufweisende Befestigungselement 3 einen fehlenden Bereich 5' des Knochengewebes 5 überbrückt, während das kürzere Befestigungselement 3a in die an die Aussenschale 1 unmittelbar anschliessende, in einem intakten Bereich des Knochengewebes 5 angebrachte Verankerungsbohrung 6 ragt.

Die Knochenschraube 7 - oder ein entsprechender Knochennagel - kann durch eine in die betreffende Führungsbohrung 11 einsetzbare Führungshülse 21 in das Knochengewebe 5 eingebracht werden. Durch die Verschlussschrauben 13 können die von Befestigungselementen 3, 3a und 4 freien Führungsbohrungen 11 gegen das Knochengewebe 5 abgedeckt werden.

Zur Vorbereitung der Implantation der künstlichen Gelenkspfanne wird im vorbestimmten Beckenbereich eine im Bausatz enthaltene Hilfsvorrichtung in Form einer Manipulierpfanne 23 befestigt, welche in ihren Aussenmassen der in einem späteren Verfahrensschritt in den Implantationsbereich einzusetzender Aussenschale 1 entspricht und welche als Bohr- bzw. Fräslehre ausgebildet ist. Entsprechend der Darstellung nach Fig. 3 ist die Manipulierpfanne 23 ebenfalls in Form einer hohlen Halbkugel sowie mit Durchgangsbohrungen 11' ausgeführt, deren Anordnung derjenigen der Führungsbohrungen 11 in der zu implantierenden Aussenschale 1 entspricht. Die Durchgangsbohrungen 11', die je zur Führung eines Bohr- bzw. Fräswerkzeugs bestimmt sind, weisen je einen Durchmesser D1 auf, der gleich oder grösser ist als der Durchmesser D der Stützplatten 17. Die Manipulierpfanne 23 kann mit einer wesentlich grösseren Wandstärke ausgeführt sein als die Aussenschale 1, wodurch eine winkelgetreue Führung des Werkzeugs gewährleistet werden kann. Die Manipulierpfanne 23 kann in bekannter Weise, z.B. durch eine im Bereich ihres Pols 8, in das Knochengewebe 5 einführbare, nicht dargestellte Knochenschraube, im Implantationsbereich um die Polachse 16 drehbar befestigt und in einer Position fixiert werden, in welcher zwei passende Durchgangsbohrungen 11' gegen die zur Aufnahme der Befestigungselemente 3 und 3a vorgesehenen Beckenpartien orientiert sind. Durch das in diese Durchgangsbohrungen 11' einführbare Bohr- bzw. Fräswerkzeug werden hierauf jeweils die Verankerungsbohrungen 6 zur Aufnahme der Befestigungselemente 3 und 3a sowie stirnseitige Anpressflächen für die Stützplatten 17 in das Knochengewebe 5 gefräst. In entsprechender Weise kann durch eine der Durchgangsbohrungen eine zur Aufnahme der Knochenschraube 7 bestimmte Bohrung in das Knochengewebe 5 eingebracht werden.

Nach Anbringung der Bohrungen werden deren Tiefen T gemessen, worauf die Manipulierpfanne 23 entfernt werden kann. Während die Manipulierpfanne 23 entfernt wird, kann durch Hilfspersonal die später einzusetzende Aussenschale 1 mit den an ihr unter den Raumwinkeln A und B der Verankerungsbohrungen 6 anzubringenden Befestigungselementen 3 und 3a bestückt werden, indem je ein aus dem bereitgestellten Vorrat ausgewählter Stützteil 18 - beim dargestellten Beispiel mindestens ein Teilstück 18b - passender Länge mit der Stützplatte 17 verbunden, von aussen her in die betreffende Führungsbohrung 11 der Aussenschale 1 eingeführt und innerhalb der Aussenschale 1 mit dem Kopfteil 20 zum Befestigungselement 3 bzw. 3a verbunden wird, dessen Einbaulänge der gemessenen Tiefe T der betreffenden Verankerungsbohrung 6 entspricht. Während dieser Operationsphase können auch die Verschlussschrauben 13 in den von Befestigungselementen 3, 3a und 4 freien Führungsbohrungen 11 angebracht werden.

Vor dem Einsetzen der Aussenschale 1 in den Implantationsbereich werden die Befestigungselemente 3 und 3a gegen die Mitte der Aussenschale 1 hin je in eine in Fig. 1 strichpunktiert dargestellte Stellung 3' bzw. 3a' eingezogen, in der die zugehörige Stützplatte 17 an der Aussenseite der Aussenschale 1 anliegt. Die so vorbereitete Aussenschale 1 kann hierauf als zusammenhängende, kompakte Baueinheit dem Chirurgen übergeben und von diesem ohne weitere Manipulation in das vorbereitete Knochenbett eingesetzt werden, worauf die Befestigungselemente 3 und 3a in die Verankerungsbohrungen 6 eingeführt und durch Einschrauben der Kopfteile 20 in die Aussenschale 1 mit dieser fest verbunden werden. Hierauf kann, falls erforderlich, die Knochenschraube 7 in die entsprechende Bohrung des Knochengewebes 5 eingeschraubt werden.

In einem weiteren Schritt kann die Innenseite der Aussenschale 1 auf vorstehende Teile überprüft und - nach Behebung allfälliger Abweichungen von der vorbestimmten Innenform - schliesslich die Innenschale 2 in die Aussenschale 1 eingesetzt werden.

## Patentansprüche

1. Bausatz für eine künstliche Gelenkpfanne, insbesondere eine Hüftgelenkpfanne, mit den folgenden Merkmalen:
- Eine metallische Aussenschale (1), welche in unterschiedlichen Raumwinkeln (A, B, C, E) angebrachte, je mit einem Gewinde (11a) versehene Führungsbohrungen (11) aufweist;
- eine in die Aussenschale (1) einsetzbare Innenschale (2);
- mindestens ein an der Aussenschale (1) befestigbares, zum Einführen in eine im Knochengewebe (5) ausgebildete Verankerungsbohrung (6) bestimmtes Befestigungselement (3, 3a), welches einen von der Innenseite der Aussenschale (1) in eine der Führungsbohrungen (11) einführbaren Kopfteil (20, 22) aufweist,
- wobei das Befestigungselement (3, 3a) in Form eines in der Verankerungsbohrung (6) abstützbaren Sockels ausgebildet ist, der eine in die Verankerungsbohrung (6) einführbare Stützplatte (17) aufweist, welche mit einem Durchmesser (D) ausgeführt ist, der grösser ist als der Durchmesser (d₁) der betreffenden Führungsbohrung (11) und welche über einen stengelartigen Stützteil (18), der mit einem Durchmesser (d) ausgeführt ist, der kleiner ist als der Durchmesser (d₁) der Führungsbohrung (11), mit dem Kopfteil (20) lösbar verbunden ist;
- eine durch eine der Aussenschale hinsichtlich deren Aussenabmessungen entsprechende, zum Vorbereiten des die Aussenschale (1) aufnehmenden Bereichs des Knochengewebes (5) bestimmte Manipulierpfanne (23), welche mit unter den Raumwinkeln (A,B,C,E) der Führungsbohrungen (11) der Aussenschale (1) angeordneten Durchtrittsöffnungen (11') versehen ist, deren Durchmesser (D₁) je dem Durchmesser (D) einer der Stützplatten entsprechen.

2. Bausatz nach Anspruch 1, dadurch gekennzeichnet, dass zwischen der Stützplatte (17) und dem Kopfteil (20) mindestens eine Schraubverbindung (18c) vorgesehen ist.

3. Bausatz nach Anspruch 1 oder 2, dadurch gekennzeichnet, dass der Stützteil (18) aus mindestens zwei der Länge nach lösbar zusammensetzbaren Teilstücken (18a, 18b) gebildet ist.

4. Bausatz nach Anspruch 3, dadurch gekennzeichnet, dass er für mindestens eines der Teilstücke (18a, 18b) des Stützteils (18) einen bereitstellbaren Vorrat solcher Teilstücke (18a, 18b) in unterschiedlichen Längen (L) enthält.

5. Bausatz nach einem der vorangehenden Ansprüche, dadurch gekennzeichnet, dass mindestens eine der von Befestigungselementen (3, 3a) freien Führungsbohrungen (11) der Aussenschale (1) mit einem in diese Führungsbohrung (11) einsetzbaren Abdeckteil, z.B. einer Verschlussschraube (13), versehen ist.

## Claims

1. A kit for an artificial joint cup, especially an acetabular cup, having the following features:
- a metallic outer shell (1), having guide bores (11) each provided with a thread (11a), mounted at differing solid angles (A,B,C,E)
- an inner shell (2) insertable into outer shell (1)
- at least one securing element (3,3a), secured to outer shell (1) intended for introduction into an anchorage bore (6) constructed in the bone tissue (5), which has a head part (20,22) which can be introduced from the inner side of outer shell (1) into one of the guide bores (11),
- whereby securing element (3,3a) is constructed in the form of a supporting base in the anchorage bore (6), having a support plate (17) which can be introduced into anchorage bore (6) which is executed having a diameter (D) which is greater than diameter (d₁) of the relevant guide bore (11) and which is releasably connected with head part (20) via a stalk-like support part (18), which is executed having a diameter (d) which is smaller than diameter (d₁) of guide bore (11),
- a manipulating cup (23) defined for preparation of the area of bone tissue (5) incorporating outer shell (1), which corresponds to the outer shell in respect of its outer measurements, which is provided with passage openings (11') arranged at the solid angles (A,B,C,E) of guide bores (11) of outer shell (1), whose diameters (D₁) correspond respectively to diameter (D) of one of the support plates.

2. A kit according to claim 1, characterised in that at least a screw connection (18c) is provided between support plate (17) and head part (20).

3. A kit according to claims 1 or 2, characterised in that support part (18) is constructed from at least two parts (18a,18b) which can be fitted together and which are longitudinally releasable.

4. A kit according to claim 3, characterised in that it contains a preparable store of such parts (18a,18b) in differing lengths (L) for at least one of the parts (18a,18b) of support part (18).

5. A kit according to any of the preceding claims, characterised in that at least one of the guide bores (11) of outer shell (1) free from securing elements (3,3a), is provided with a cover part e.g. a locking screw (13) which can be inserted into this guide bore (11).

## Revendications

1. Jeu de pièces pour une cupule articulaire artificielle, notamment une cupule acétabulaire, avec les caractéristiques suivantes :
- une coque extérieure métallique (1) qui présente des perçages de guidage (11) disposés à des angles solides différents (A, B, C, E), pourvus chacun d'un filetage (11a);
- une coque intérieure (2) pouvant être placée dans la coque extérieure (1);
- au moins un élément de fixation (3, 3a) pouvant être fixé à la coque extérieure (1), prévue pour l'insertion dans un perçage d'ancrage (6) réalisé dans le tissu osseux (5), qui présente une partie de tête (20, 22) insérable depuis le côté intérieur de la coque extérieure (1) dans l'un des perçages de guidage (11);
- où l'élément de fixation (3, 3a) est réalisée sous la forme d'un socle prenant appui dans le perçage d'ancrage (6) qui présente une plaque d'appui (17) insérable dans le perçage d'ancrage (6), qui est réalisé avec un diamètre (D) qui est plus grand que le diamètre (d₁) du perçage de guidage concerné (11) et qui est relié amoviblement par une partie d'appui (18) en forme de tige, qui est réalisée avec un diamètre (d) qui est plus petit que le diamètre (d₁) du perçage de guidage (11), à la partie de tête (20);
- une cupule de manipulation (23) correspondant à la coque extérieure quant à ses dimensions extérieures, destinée à préparer la zone du tissu osseux (5) recevant la coque extérieure (1), qui est pourvue d'ouvertures traversantes (11') disposées selon les angles solides (A, B, C, E) des perçages de guidage (11) de la coque extérieure (1), dont les diamètres (D₁) correspondent chacun au diamètre (D) d'une des plaques d'appui.

2. Jeu de pièces selon la revendication 1, caractérisé en ce qu'il est prévu entre la plaque d'appui (17) et la partie de tête (20) au moins un assemblage par vissage (18c).

3. Jeu de pièces selon la revendication 1 ou 2, caractérisé en ce que la partie d'appui (18) est formée par au moins deux pièces partielles (18a, 18b) pouvant être assemblées amoviblement en longueur.

4. Jeu de pièces selon la revendication 3, caractérisé en ce qu'il comporte pour au moins l'une des pièces partielles (18a, 18b) de la partie d'appui (18) une réserve pouvant être mise à disposition de telles pièces partielles (18a, 18b) en des longueurs différentes (L).

5. Jeu de pièces selon l'une des revendications précédentes, caractérisé en ce qu'au moins l'un des perçages de guidage (11) exempts des éléments de fixation (3, 3a) de la coque extérieure (1) est pourvu d'une partie de recouvrement insérable dans ce perçage de guidage (11), par exemple d'une vis de fermeture (13).
